# EUROPEAN PATENT APPLICATION

(11) **EP 3 868 392 A1**
(43) Date of publication of application: **25.08.2021**
(21) Application number: 19873093.9
(22) Date of filing: 17.10.2019
(51) Int. Cl.: A61K 38/16, A61P 29/00, A61K 8/64, A61Q 19/00

(54) **PHARMACEUTICAL COMPOSITION FOR PREVENTING OR TREATING INFLAMMATORY DISEASES**

(30) Priority: 19.10.2018 KR 20180124897
(71) Applicant: Eyegene Inc., Seoul 07528 (KR)
(72) Inventor: CHO, Yang Je, Seoul 04423 (KR); NAM, Hee Jin, Seoul 02844 (KR)
(74) Representative: Hiebl, Inge Elisabeth
(86) International application number: PCT/KR2019/013625
(87) International publication number: WO 2020/080836

(57) **Abstract**

The present invention provides a pharmaceutical composition having an excellent anti-inflammatory effect, thereby enabling effective prevention or treatment of inflammatory diseases such as reperfusion injury, periodontitis, arthritis, pressure sore inflammation, wound inflammation or dermatitis, and provides an anti-inflammatory cosmetic composition to be used as a raw material of cosmetic products that can be used for various inflammatory skin diseases.

## Description

### Technical Field

The present invention relates to a pharmaceutical composition for preventing or treating inflammatory diseases having an excellent anti-inflammatory effect.

### Background Art

Inflammation is a defense response of the body to prevent damage to body tissue by external physical stimulation, chemical stimulation, for example, contact of various allergens, or the invasion of microorganisms such as bacteria, fungi or viruses.

Inflammatory signals are produced through the cyclooxygenase (COX) pathway or the lipoxygenase (LOX) pathway, and produce prostaglandins, thromboxane, and the like. When the inflammatory signal is transmitted, various changes occur in the body. One change is a phenomenon in which the blood vessels expand in the area where inflammation is required, thus resulting in intensive supply of blood cells necessary for the inflammatory reaction, such as neutrophils. However, an abnormally excessive bodily defense response may cause various inflammatory diseases. In an attempt to prevent this phenomenon, drugs capable of suppressing excessive inflammatory reactions by blocking the inflammatory signaling pathways through inhibition of enzymes associated with the inflammatory signaling pathways (e.g., COX-1, COX-2, 5-LOX, 12-LOX, etc.) are being developed.

Inflammation is classified into acute inflammation (immediate reaction, nonspecific reaction, several days to several weeks), chronic inflammation (delayed reaction, specific reaction, more than several weeks), and subacute inflammation (intermediate stage of acute inflammation and chronic inflammation, characterized by mixed products of polymorphonuclear cells and mononuclear cells) depending on the duration of the reaction.

In addition, factors that cause or mediate inflammation include, in addition to peptide factors, lipid factors such as prostaglandin, leukotriene and platelet-activating factors, inflammatory factor synthases, free radicals such as NO (nitric oxide), various types of cell adhesion molecules, immune system factors and coagulation factors.

The mechanism of inflammation known to date is that cell damage is caused by external biological factors (bacteria, viruses, parasites), physical factors (mechanical stimulation, heat, radiation, electricity), chemical factors and the like, causing the release of histamine and kinin, and then vascular dilation, increased capillary permeability, and aggregation of macrophages to the inflammatory sites, thus resulting in phenomena such as increased blood flow to the infected site, swelling, movement of immune cells and antibodies, pain, and fever.

Currently used therapeutic agents for inflammation include synthetic drugs such as ibuprofen, antihistamines, steroids, cortisone, immunosuppressants and immune boosters, but they have limitations of only temporary therapeutic effects or simple symptomatic relief, or cause many side effects such as hypersensitivity and immune system deterioration, and are thus difficult to use to fundamentally treat inflammation.

Therefore, in an attempt to effectively alleviate inflammation, research has been recently conducted on a substance capable of inhibiting the expression of inflammation-related proteins. However, issues associated with side effects have arisen in anti-inflammatory substances developed as a result of such research. Drugs for suppressing inflammation based on various mechanisms including nonsteroidal anti-inflammatory drugs (NSAIDs) and steroidal anti-inflammatory drugs (SAIDs) have been developed. However, these drugs have some side effects and do not fundamentally inhibit the inflammatory response, so there is still a need for more effective, safe and economical drugs. For example, nonsteroidal anti-inflammatory drugs used for the treatment of chronic inflammatory diseases such as acute or rheumatoid arthritis are known to cause side effects such as gastrointestinal disorders by inhibiting a COX-2 enzyme as well as a COX-1 enzyme.

### Disclosure

### Technical Problem

It is one object of the present invention to provide a pharmaceutical composition and a cosmetic composition having an excellent anti-inflammatory effect.

### Technical Solution

In accordance with one aspect of the present invention, the above and other objects can be accomplished by the provision of a pharmaceutical composition for preventing or treating an inflammatory disease comprising a polypeptide consisting of the sequence of SEQ ID NO: 1.

The inflammatory disease may include at least one selected from the group consisting of neuroinflammation, reperfusion injury, periodontitis, arthritis, dermatitis, wound inflammation, bedsore inflammation, spondylitis, urethritis, cystitis, nephritis, pyelonephritis, vasculitis, rhinitis, sore throat, tonsillitis, acute pain, and inflammatory bowel disease.

In accordance with another aspect of the present invention, there is provided an anti-inflammatory cosmetic composition comprising a polypeptide consisting of the sequence of SEQ ID NO: 1 .

### Advantageous Effects

The present invention provides a pharmaceutical composition having an excellent anti-inflammatory effect to effectively prevent or treat inflammatory diseases such as reperfusion injury, periodontitis, arthritis or dermatitis, and provides an anti-inflammatory cosmetic composition useful as a raw material for cosmetic products for various inflammatory skin diseases.

### Brief Description of Drawings

FIG. 1 shows data comparing the expression levels of TNF-α between an untreated control group, a control group treated only with LPS, an experimental group treated with the peptide of the present invention along with LPS, and an experimental group treated only with the peptide of the present invention in Example 1.
FIG. 2 shows the expression levels of IL-8, CXCL1 and CXCL10 between the control group treated with LPS alone and the group treated with a combination of LPS and the peptide of the present invention in Example 2.
FIG. 3 is a schematic diagram showing a stained heart tissue site used for immunohistochemistry (IHC) in Example 3.
FIG. 4 shows the result of measuring the expression level of eNOS in Groups 1 to 4 in Example 3 .
FIG. 5 shows the result of measuring the expression level of TNF-α in Groups 1 to 4 in Example 3 .
FIG. 6 shows the result of measuring the expression level of CD68 (macrophage) in Groups 1 to 4 in Example 3.
FIG. 7 shows the result of measuring the expression level of Ly-6B (neutrophil) in Groups 1 to 4 in Example 3.
FIG. 8 shows the result of determining the survival probability of each group through observation of general symptoms after ligation, after reperfusion, and during the breeding period after production in Example 4.
FIGS. 9 and 10 show results of measuring EF and FS values using echocardiography in Example 4.
FIG. 11 shows the result of measuring the infarct size and fibrosis degree of the myocardial tissue in Example 4.

### Detailed Description and Exemplary Embodiments

Hereinafter, the present invention will be described in detail.

The present invention provides a pharmaceutical composition for preventing or treating an inflammatory disease comprising a polypeptide consisting of the sequence of SEQ ID NO: 1.

As used herein, the term "treatment" refers to an approach to obtain beneficial or desirable clinical results. For the purposes of the present invention, beneficial or desirable clinical results include, but are not limited to, alleviation of symptoms, reduction of disease range, stabilization of disease condition (i.e., prevention of exacerbation), delay or decrease in disease progression, amelioration or temporal alleviation and alleviation of disease condition (partially or entirely), and whether or not detection is possible. Further, the term "treatment" may refer to increasing the survival probability compared to the survival probability that is expected when not receiving treatment. Treatment encompasses both therapeutic treatment and prophylactic or preventative measures. Such treatment includes treatment required for a disorder to be prevented and treatment required for a disorder that has already developed.

As used herein, the term "prevention" refers to any action that suppresses or delays the onset of related diseases. It will be obvious to those skilled in the art that the composition of the present invention can prevent a related disease when administered before initial symptoms or onset of the disease.

The polypeptide consisting of the sequence of SEQ ID NO: 1 has excellent anti-inflammatory efficacy. For example, the polypeptide effectively reduces the expression levels of eNOS, TNF-α, CD68 and Ly-6B (neutrophil), which are known as inflammatory markers, thereby exhibiting the effect of preventing or treating various inflammatory diseases.

The polypeptide of the present invention can be chemically synthesized. When the polypeptide is prepared by chemical synthesis, it can be prepared by chemical synthesis (Creighton, Proteins; Structures and Molecular Principles, W. H. Freeman and Co., NY, 1983) well known in the art. Representative methods include, but are not limited to, liquid or solid phase synthesis, fragment condensation, F-MOC or T-BOC chemical methods (Chemical Approaches to the Synthesis of Peptides and Proteins, Williams et al., Eds., CRC Press, Boca Raton Florida, 1997; A Practical Approach, Atherton & Sheppard, Eds., IRL Press, Oxford, England, 1989) .

In addition, the polypeptide of the present invention may be prepared by a genetic engineering method, such as the following method, which is not limiting. First, a DNA sequence encoding the polypeptide is produced in accordance with a conventional method. DNA sequences can be produced by PCR amplification using appropriate primers. Alternatively, DNA sequences can be also synthesized by standard methods known in the art, for example, using an automatic DNA synthesizer (e.g., sold by Biosearch or Applied Biosystems). The produced DNA sequence is inserted into a vector containing one or more expression control sequences (e.g., promoters, enhancers, etc.) that are operably linked to the DNA sequence and control the expression of the DNA sequence, and host cells are transformed with the resulting recombinant expression vector. The resulting transformant is cultured under a medium and conditions suitable to allow the DNA sequence to be expressed, and the substantially pure polypeptide encoded by the DNA sequence is recovered from the culture product. The recovery may be performed using a method known in the art (e.g., chromatography) . The term "substantially pure polypeptide" described above means that the polypeptide according to the present invention does not substantially contain any other protein derived from a host. For the genetic engineering method for synthesizing the polypeptide of the present invention, reference can be made to the following literature: Maniatis et al., Molecular Cloning; A laboratory Manual, Cold Spring Harbor laboratory, 1982; Sambrook et al., Molecular Cloning: A Laboratory Manual, ColdSpring Harbor Press, N.Y., Second(1998) and Third(2000) Edition; Gene Expression Technology, Method in Enzymology, Genetics and Molecular Biology, Method in Enzymology, Guthrie & Fink (eds.), Academic Press, San Diego, Calif, 1991; and Hitzeman et al., J.Biol. Chem., 255:12073-12080, 1990.

The pharmaceutical composition may be formulated and used in the form of an oral formulation such as a powder, granule, tablet, capsule, suspension, emulsion, syrup or aerosol, an external preparation, a suppository, or a sterile injectable solution in accordance with a conventional method, but is not limited thereto.

The carrier, excipient or diluent which may be contained in the pharmaceutical composition comprising the polypeptide according to the present invention may include, but is not limited to, lactose, dextrose, sucrose, dextrin, maltodextrin, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate and mineral oil. In the case of formulation, a typically used diluent or excipient, such as a filler, an extender, a binder, a wetting agent, a disintegrant or a surfactant, is used, but is not limited thereto.

Solid formulations for oral administration may include, but are not limited to, tablets, pills, powders, granules, capsules and the like, and solid formulations may be prepared by mixing at least one excipient, for example, starch, calcium carbonate, sucrose, lactose, gelatin, or the like. In addition to simple excipients, lubricants such as magnesium stearate and talc are also used.

Liquid formulations for oral administration may be suspensions, oral liquids and solutions, emulsions, syrups and the like, and may include various excipients such as wetting agents, sweeteners, fragrances, preservatives and the like, in addition to water and liquid paraffin, which are simple diluents that are commonly used. Formulations for parenteral administration may be sterilized aqueous solutions, non-aqueous solutions, suspensions, emulsions, freeze-dried preparations and suppositories. Examples of the non-aqueous solutions and suspensions include propylene glycol, polyethylene glycol, vegetable oil such as olive oil, injectable esters such as ethyl oleate, and the like. Examples of the suppository base include Witepsol, macrogol, Tween 61, cacao butter, laurin butter, glycerogelatin and the like.

The composition of the present invention acts as an anti-inflammatory agent and can be used for prevention and treatment of an inflammatory disease such as wound inflammation, bedsore inflammation, dermatitis including atopic dermatitis, neuritis, spondylitis, urethritis, cystitis, nephritis, pyelonephritis, vasculitis, rhinitis, periodontitis, arthritis, sore throat, tonsillitis, acute pain, reperfusion injury or inflammatory bowel diseases. Any inflammatory disease can be used without particular limitation.

The present invention also provides an anti-inflammatory cosmetic composition comprising a polypeptide consisting of the sequence of SEQ ID NO: 1.

When the composition of the present invention is prepared as a cosmetic composition, the composition of the present invention may comprise, in addition to the polypeptide, components commonly used in cosmetic compositions, for example, conventional adjuvants such as antioxidants, stabilizers, solubilizers, vitamins, pigments and fragrances, and carriers.

Examples of products to which the composition can be added include, but are not limited to, cosmetics such as astringent, softening lotion, nutrient lotion, various creams, essences, packs and foundations, and cleansers, face wash, soap, treatments, beauty lotions, and the like.

Specific formulations of the cosmetic composition of the present invention include formulations such as skin lotion, skin softener, skin toner, astringent, lotion, milk lotion, moisture lotion, nutrition lotion, massage cream, nutrition cream, moisture cream, hand cream, essence, nutrition essence, pack, soap, shampoo, cleansing foam, cleansing lotion, cleansing cream, body lotion, body cleanser, emulsion, lipstick, makeup base, foundation, press powder, loose powder, and eye shadow.

The composition may be formulated by stabilizing the polypeptide by incorporating the polypeptide in the nanoliposome. When the polypeptide is contained in the nanoliposome, the components of the polypeptide are stabilized, problems such as precipitation and transformation can be solved during formulation, the solubility and transdermal absorption of the component can be increased, and the efficacy expected from the polypeptide can be maximized.

The present invention also provides an anti-inflammatory health functional food comprising a polypeptide consisting of the sequence of SEQ ID NO: 1.

The health functional food of the present invention can be produced and processed in the form of a tablet, capsule, powder, granule, liquid, pill, or the like for antioxidant or anti-inflammatory purposes.

The health functional food of the present invention refers to a food produced and processed using raw materials or ingredients having useful functions for the human body pursuant to the Health Functional Food Act No. 6727. A health functional food is a substance taken for the purpose of controlling nutrients or obtaining beneficial effects for health purposes such as physiological effects with regard to the structure and function of the human body.

The health functional food of the present invention may comprise an ordinary food additive, and whether or not the food additive is suitable is determined based on the criteria and standards for the target item pursuant to general rules and general test methods of food additive codes approved by the Food and Drug Administration, unless otherwise specified.

Examples of items listed in the food additive codes include, but are not limited to, chemical synthetic compounds such as ketones, glycine, calcium citrate, nicotinic acid and cinnamic acid, natural additives such as persimmon color, licorice extract, crystalline cellulose, kaoliang color and guar gum, and mixed agents such as sodium L-glutamate, alkali additives for noodles, preservatives and tar colors.

For example, a tablet-type health functional food may be produced by granulating a mixture obtained by mixing the polypeptide with an excipient, a binder, a disintegrant, and other additives, by a conventional method, adding a lubricant thereto and then conducting compression molding, or by directly conducting compression molding on the mixture. In addition, the tablet-type health functional food may contain a flavor or the like, as necessary.

Among capsule-type health functional foods, hard capsules can be prepared by filling a conventional hard capsule base with a mixture of the polypeptide and additives such as excipients, and soft capsules can be prepared by filling a capsule base such as gelatin with a mixture of the polypeptide and additives such as excipients. The soft capsule may contain a plasticizer such as glycerin or sorbitol, a colorant, a preservative, or the like, if necessary.

A pill-type health functional food can be prepared by molding a mixture of the polypeptide with an excipient, binder, disintegrant, or the like by a conventionally known method, and can be coated with white sugar or another coating agent as needed, or can also be surface-coated with a material such as starch or talc.

A granule-type health functional food can be prepared in a granular form using a mixture of the polypeptide with an excipient, binder, disintegrant, or the like by a conventionally known method, and may contain a perfume, flavoring agent, or the like if necessary.

Examples of the health functional food include beverages, meat, chocolate, foods, confectioneries, pizza, ramen, other noodles, gum, candy, ice cream, alcoholic beverages, vitamin complexes, health supplements and the like.

Hereinafter, the present invention will be described with reference to examples in detail.

Hereinafter, the peptide consisting of the sequence of SEQ ID NO: 1 may be abbreviated as "peptide of the present invention".

### Example 1: Determination of TNF-α expression inhibitory ability

### (1) Experiment method

THP-1 (human monocytic cell line) cells in culture, were pre-treated with the peptide of the present invention (F1701) for 30 minutes and then treated with LPS (1 µg/mL), followed by allowing a reaction to proceed for 24 hours. After the reaction, the medium was collected, the remaining cells were removed, and the expression level of TNF-α was measured using a Duoset® ELISA Human TNF-α (DY210-05, R&D Systems).

### (2) Experimental result

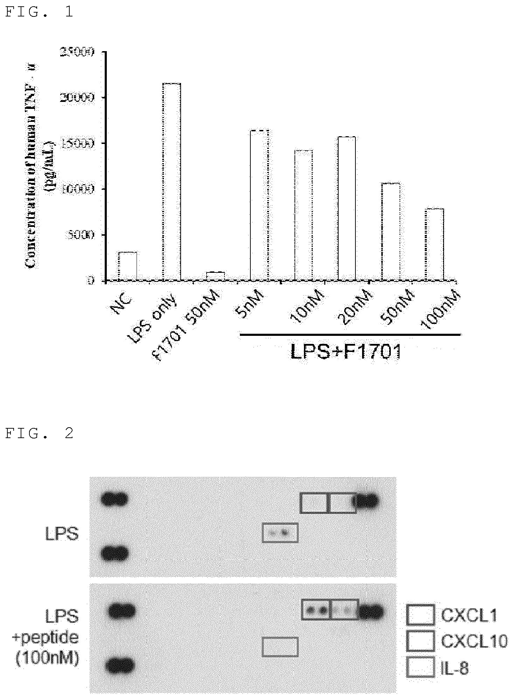

Data comparing the expression levels of TNF-α between the untreated control group, the control group treated only with LPS, the experimental group treated with the peptide of the present invention along with LPS, and the experimental group treated only with the peptide of the present invention can be seen from FIG. 1. As can be seen from FIG. 1, treatment with the peptide of the present invention alone can more effectively inhibit the expression of TNF-α compared to the untreated control group, and treatment with the LPS along with the peptide of the present invention can effectively inhibit the expression of TNF-α in a concentration-dependent manner, unlike the control group treated only with LPS. This is considered to be a meaningful experimental result suggesting that a therapeutic effect as well as a preventive effect on various inflammatory diseases can be obtained by inhibiting the expression of the inflammatory disease marker using the peptide of the present invention.

### Example 2. Determination of control ability of IL-8, CXCL1 and CXCL10 expression levels

### (1) Experimental method

THP-1 (human monocytic cell line) in culture was seeded at 1.0X10⁶/well in a 24-well plate and cultured for 16 hours. The THP-1 cells were pre-treated with the peptide of the present invention (F1701) at a final concentration of 100 nM for 30 minutes, followed by treatment with LPS (1 µg/mL) and culture for 24 hours. Sample preparation was performed by collecting the culture medium, removing the remaining cells through centrifugation, and then using only the supernatant. The expression levels of cytokines before and after treatment were compared using the Proteome Profiler Human Cytokine Array kit (R&D) system.

### (2) Experimental result

The expression levels of IL-8, CXCL1, and CXCL10 in the control group treated with LPS alone, and the group treated with LPS along with the peptide of the present invention can be seen from FIG. 2. It can be seen from FIG. 2 that the group treated with the peptide of the present invention exhibited a decreased expression level of IL-8 and increased expression levels of CXCL1 and CXCL10 compared to the control group. This is considered to be data proving that the peptide of the present invention reduces the expression level of IL-8, which is an inflammatory marker, and increases the expression levels of CXCL1 and CXCL10, thereby activating a mechanism for healing wounds and as a meaningful result identifying that the peptide of the present invention can be effective in preventing or treating inflammatory diseases and healing various wounds.

### [Example 3. Evaluation of inhibitory activity against expression level of reperfusion injury-related marker gene

### (1) Experimental method

### 1) Experimental system and test group

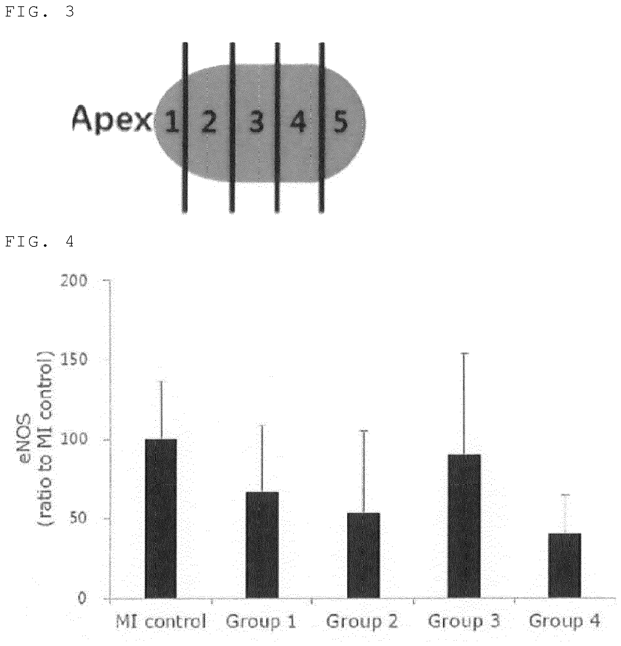

Male Sprague-Dawley (SD) rats were used as the subject of the *in vivo* experiment, and 176 pieces of cardiac tissue were used. Among them, the dyed heart tissue sites used for IHC were sites 2 and 3 in the schematic diagram of FIG. 3.

Paraffin blocks and slides were produced as experimental groups, and all experimental groups were treated with the peptide of the present invention once after ligation and once after reperfusion (twice in total in common). Specific experimental group information is shown in Table 1 below.

**[Table 1]**

| Group | Dosage (µg/kg/each) | Frequency of administration | eNOS (number of rats) | TNF-α (number of rats) | Macrophage (number of rats) | Neutrophil (number of rats) |
|---|---|---|---|---|---|---|
| Sham control | 0 | administration twice in common | 3 | 3 | 3 | 3 |
| MI/R control | 0 | administration twice in common | 10 | 10 | 10 | 10 |
| Group 1 | 150 | administration twice in common | 8 | 8 | 8 | 8 |
| Group 2 | 150 | administration twice in common + additional administration once | 8 | 8 | 8 | 8 |
| Group 3 | 150 | administration twice in common + additional administration twice | 7 | 7 | 7 | 7 |
| Group 4 | 150 | administration twice in common + additional administration three times | 8 | 8 | 8 | 8 |

### 2) Experimental method

Immunohistochemistry (IHC) was performed using cardiac tissue slides. The tissue was imaged with a slide scanner, and the size of the area of expression was measured using a program in the Leica application suite to analyze the expression ratio.

### 3) Statistical analysis

The result of the efficacy evaluation was obtained by comparing the mean and standard deviation calculated between experimental groups. The statistical method that was used was a one-way ANOVA test, and significance between groups was determined based thereon. Data are expressed as mean ± SD. The result of a test item was considered significant in the case of p<0.05, and the computer program for statistics used herein was SPSS 23 (IBM, USA).

### (2) Experimental result

### 1) Measurement of eNOS expression level

The results of measuring the expression levels of eNOS in Groups 1 to 4 can be seen from FIG. 4. It can be seen that the expression level of eNOS decreases in all groups treated with the peptide of the present invention compared to the control group. In particular, Group 4 exhibits a decrease in the expression level of eNOS by more than half compared to the control group. This means that the peptide of the present invention is effective in preventing or treating reperfusion damage by reducing the expression level of eNOS in the myocardium and thus inhibiting myocardial malfunction.

### 2) Measurement of TNF-α expression level

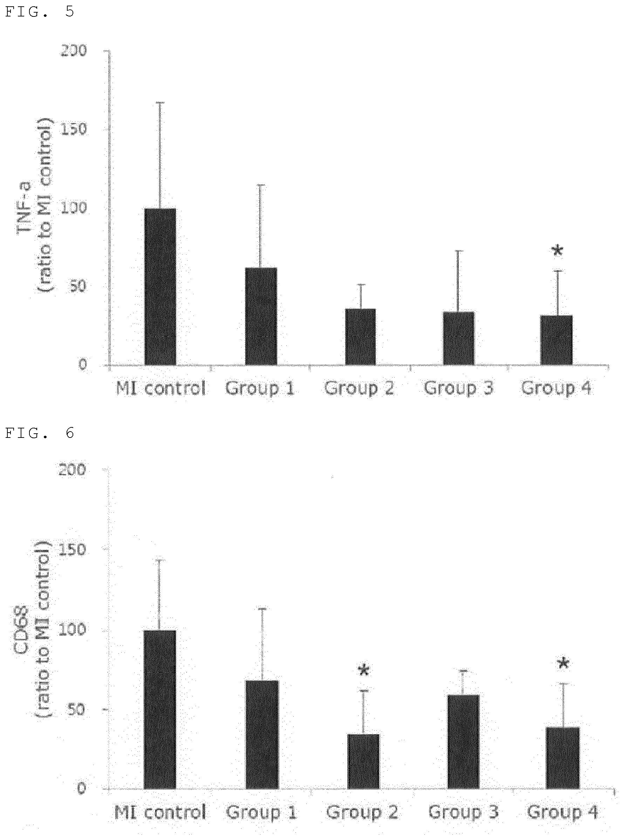

The results of measuring the expression levels of TNF-α in Groups 1 to 4 can be seen from FIG. 5. It can be seen that the expression level of TNF-α decreases in the group treated with the peptide of the present invention compared to the control group. In particular, Groups 2 to 4 exhibit a decrease in the expression level of TNF-α of about 25% compared to the control group. This means that the peptide of the present invention is effective in preventing or treating reperfusion damage by reducing the expression level of TNF-α in the myocardium and thus preventing progression to an inflammatory reaction.

### 3) Measurement of CD68 expression level

The results of measuring the expression levels of CD68 (macrophage) in Groups 1 to 4 can be seen from FIG. 6. It can be seen that the expression level of CD68 remarkably decreases in all groups treated with the peptide of the present invention compared to the control group. In particular, Groups 2 and 4 exhibit a decrease in the expression level of CD68 of about 25% compared to the control group. This means that the peptide of the present invention is effective in preventing or treating reperfusion damage by reducing the expression level of CD68 in the myocardium and thus promoting an anti-inflammatory effect.

### 4) Measurement of LY-6B expression level

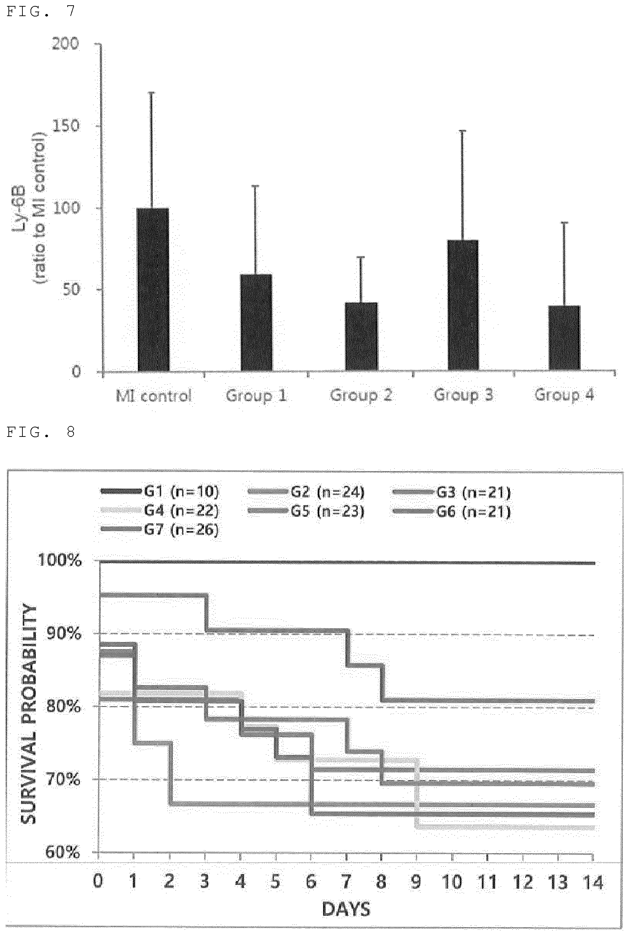

The results of measuring the expression levels of LY-6B (neutrophil) in Groups 1 to 4 can be seen from FIG. 7. It can be seen that the expression level of LY-6B remarkably decreases in all groups treated with the peptide of the present invention compared to the control group. In particular, Groups 2 and 4 exhibit a decrease in the expression level of LY-6B of about 40% compared to the control group. This means that the peptide of the present invention is effective in preventing or treating reperfusion damage by reducing the expression level of LY-6B in the myocardium and thus promoting an anti-inflammatory effect.

### Example 4. Evaluation of effectiveness of improving cardiac function on inhibition of reperfusion injury

### (1) Experimental method

As the subjects of the *in vivo* experiment, 147 male Sprague-Dawley (SD) rats (7 weeks old, 270-320g), which are widely used for cardiovascular disease efficacy evaluation, were used. After the MI/R model was produced, a control substance and a test substance were administered to each group as shown in Table 2 below. The substances were administered subcutaneously (S.C.) once immediately after ligation, once immediately after reperfusion, and twice in total. Subcutaneous administration was performed at the femoral site and in the nape of the neck from 1 day after model production.

**[Table 2]**

| Group | Information | Dosage | Number of rats | Substance administered (8 times in total) |
|---|---|---|---|---|
| G1 | Simulated control group (No MI/R model) | 18 mg/kg | 10 | D-mannitol |
| G2 | Experimental control group (MI/R model) | 18 mg/kg | 16 | D-mannitol |
| G3 | Experimental group (MI/R model) | 50 µg/kg | 15 | Peptide of present invention |
| G4 | Experimental group (MI/R model) | 150 µg/kg | 14 | Peptide of present invention |
| G5 | Experimental group (MI/R model) | 300 µg/kg | 16 | Peptide of present invention |
| G6 | Experimental group (MI/R model) | 450 µg/kg | 17 | Peptide of present invention |
| G7 | Experimental group (MI/R model) | 600 µg/kg | 17 | Peptide of present invention |

In order to determine the functions of the left ventricle, EF and FS values were measured using echocardiography before MI/R model production and on the 6th and 14th days after MI/R model production, and on the 14th day after model production, cardiac tissue slides were produced and IHC was performed. Echocardiography was conducted by measuring the ejection factor (EF) and fractional shortening (FS) using a Vevo-2100 (VisualSonics, CAN) apparatus on the 6th and 14th days after model production. For IHC, cardiac tissue was imaged with a slide scanner, and the size of the area of expression was measured using a Leica application suite program to analyze the expression ratio. The heart infarct area was measured using a modified Masson's trichrome stain kit, and the sizes of the heart area and MI area were measured using the Leica application suite program (Leica, DEU) to analyze the ratio of the MI. The result of the efficacy evaluation was obtained by comparing the mean and standard deviation calculated between experimental groups. The statistical method used was a one-way ANOVA test, and significance between groups was determined based thereon. Data are expressed as mean ± SD. The evaluation of efficacy for determining a clinical dose of the peptide of the present invention in an ischemic acute myocardial infarction model was considered to be significant in the case of p<0.05, and the computer program for statistics used herein was SPSS 23 (IBM, USA).

### (2) Experimental result

### 1) Survival probability

Referring to FIG. 8, the survival probability of each group can be determined by observing general symptoms after ligation, after reperfusion, and during the breeding period after production. The survival probability of all groups (Groups 3 to 7) administered with the peptide of the present invention during the experimental period was 80% or more until Day 3, which is much higher than that of the experimental control group (Group 2), in which survival probability fell to 75% on Day 2. In particular, it can be seen that Group 6 had much higher survival probability than the other groups, and maintained a high survival probability exceeding 80% even on Day 14. This suggests that the ability to inhibit reperfusion damage upon treatment with the peptide of the present invention at a high concentration is better than in the case of the other groups.

### 2) Evaluation of left ventricular function using echocardiography

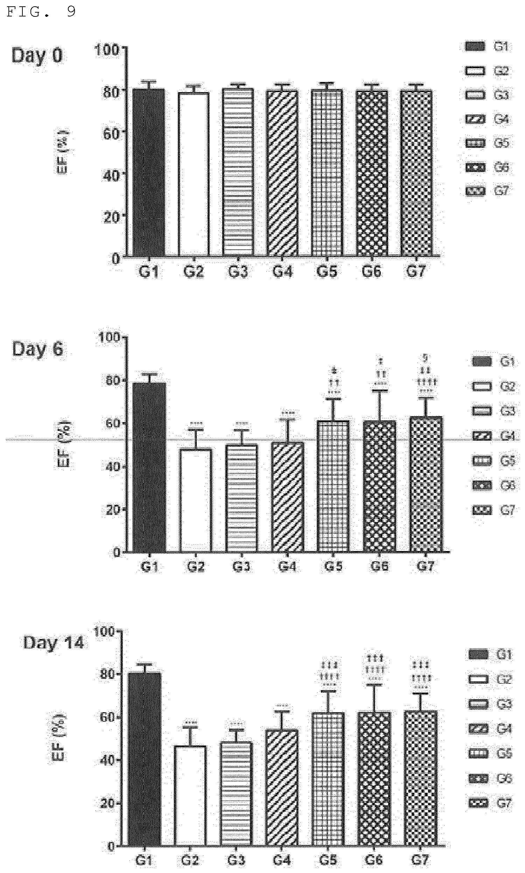

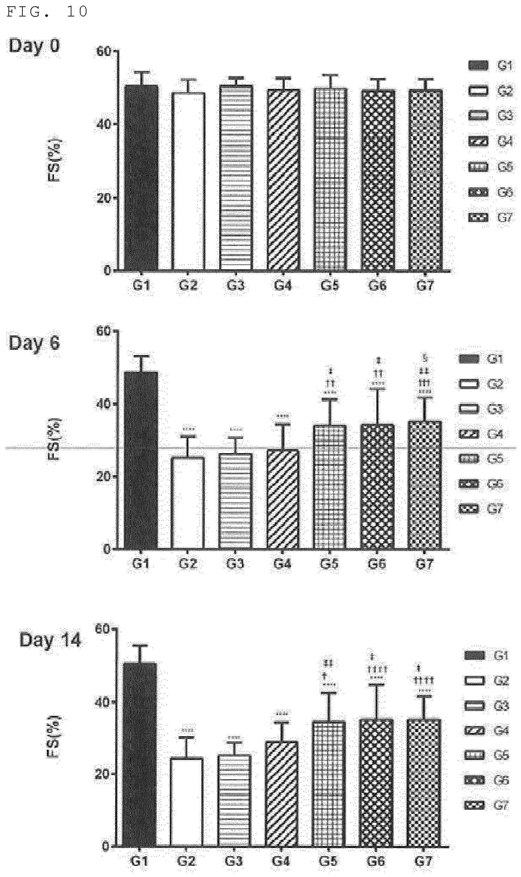

The results of measuring the EF and FS values using echocardiography before MI/R model production and 6 and 14 days after model production in order to evaluate the function of the left ventricle can be determined from Table 3 below. It can be seen that EF and FS were higher in all experimental control groups (Groups 3 to 7) compared to the control group, namely Group 2. In particular, it can be seen that Groups 5 to 7 exhibited much higher EF and FS values than those of the control experimental group. The above results suggest that treatment with the peptide of the present invention can suppress reperfusion damage and can very effectively inhibit damage to the contractile function of the left ventricle. FIGS. 9 and 10 are graphs showing the results shown in Table 3 below (*p<0.005, **p<0.001, ***p<0.0005, ****p<0.0001 versus G1), (+p<0.005, ++p<0.001, +++p<0.0005, ++++p<0.0001 versus G2), (#p<0.05, #p<0.005 versus G3), (§p<0.05 versus G4).

**[Table 3]**

| Group | EF (%) | | | FS (%) | | |
|---|---|---|---|---|---|---|
| | Day 0 | Day 6 | Day 14 | Day 0 | Day 6 | Day 14 |
| G1 | 80.1±3.9 | 78.4±4.5 | 80.1±4.4 | 50.3±4.0 | 48.7±4.4 | 50.5±4.9 |
| G2 | 78.3±3.6 | 47.9±9.0 | 46.3±8.8 | 48.6±3.6 | 25.1±6.0 | 24.2±5.9 |
| G3 | 80.4±2.2 | 49.9±6.9 | 48.1±5.8 | 50.5±2.3 | 26.3±4.4 | 25.1±3.4 |
| G4 | 79.3±3.2 | 51.0±10.6 | 53.6±8.8 | 49.4±3.3 | 27.3±7.1 | 28.8±5.7 |
| G5 | 79.7±3.5 | 60.8±10.4 | 61.5±10.5 | 49.9±3.3 | 34.0±7.3 | 34.7±7.9 |
| G6 | 79.2±3.1 | 60.5±14.4 | 61.8±12.9 | 49.3±3.1 | 34.3±9.9 | 35.2±9.5 |
| G7 | 79.3±3.0 | 62.5±9.2 | 62.3±8.5 | 49.4±3.0 | 35.2±6.6 | 35.3±6.4 |

### 3) Infarct area evaluation

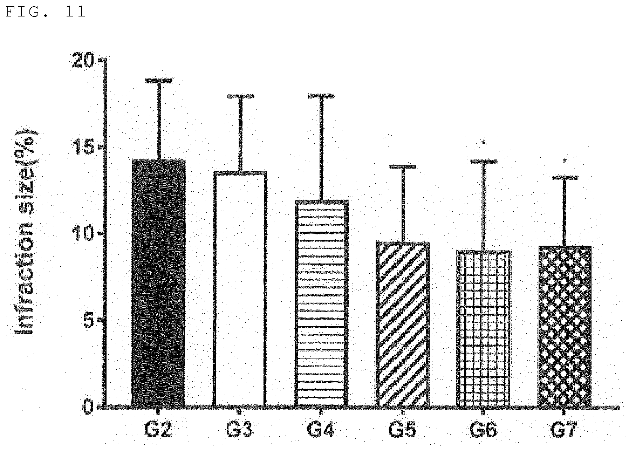

As can be seen from Table 4 below, in order to measure the infarct size and degree of fibrosis of the myocardial tissue, the heart was excised on the 14th day after the MI/R model was produced, Masson's trichrome (MT) staining was performed, five slides for each subject were produced, the ratio of the infarct area to the total area of the left ventricle was calculated, and the mean was obtained. It can be seen that the mean infarct area ratio was low in all experimental groups (Groups 3 to 7) compared to the experimental control group, namely, Group 2. In particular, it can be seen that Groups 5 to 7 exhibited much lower mean infarct area ratios than those of the experimental control group. The above results suggest that treatment with the peptide of the present invention has a remarkable effect of preventing or treating reperfusion injury by inhibiting infarction of the myocardial tissue due to reperfusion. FIG. 11 is a graph showing the results shown in Table 4 below (*p<0.05, **p<0.01, ***p<0.005 versus G2).

**[Table 4]**

| | Day 14 infarct area / Total area of left ventricle (%) |
|---|---|
| Group G2 | 14.3±4.5 |
| G3 | 13.6±4.3 |
| G4 | 11.9±6.0 |
| G5 | 9.5±4.3 |
| G6 | 9.1±5.1 |
| G7 | 9.3±3.9 |

## Claims

1. A pharmaceutical composition for preventing or treating an inflammatory disease comprising a polypeptide consisting of the sequence of SEQ ID NO: 1.

2. The pharmaceutical composition according to claim 1, wherein the inflammatory disease comprises at least one selected from the group consisting of neuroinflammation, reperfusion injury, periodontitis, arthritis, dermatitis, wound inflammation, bedsore inflammation, spondylitis, urethritis, cystitis, nephritis, pyelonephritis, vasculitis, rhinitis, sore throat, tonsillitis, acute pain and an inflammatory bowel disease.

3. An anti-inflammatory cosmetic composition comprising a polypeptide consisting of the sequence of SEQ ID NO: 1.


<110>    EYEGENE INC.

<120>    PHARMACEUTICAL COMPOSITION FOR PREVENTING OR TREATING
         INFLAMMATORY DISEASE

<130>    18P09078

<150>    KR 10-2018-0124897
<151>    2018-10-19

<160>    1

<170>    KoPatentIn 3.0

<210>    1
<211>    58
<212>    PRT
<213>    Artificial Sequence

<220>
<223>    Polypeptide sequence 1


<400>    1
Thr Cys Gln Leu Arg Pro Gly Ala Gln Cys Ala Ser Asp Gly Pro Cys
  1               5                  10                  15

Cys Gln Asn Cys Gln Leu Arg Pro Ser Gly Trp Gln Cys Arg Pro Thr
             20                  25                  30

Arg Gly Asp Cys Asp Leu Pro Glu Phe Cys Pro Gly Asp Ser Ser Gln
         35                  40                  45

Cys Pro Pro Asp Val Ser Leu Gly Asp Gly
     50                  55